(19) Europäisches Patentamt
European Patent Office
Office européen des brevets

(11) EP 0 935 959 B1

(12) EUROPÄISCHE PATENTSCHRIFT

(45) Veröffentlichungstag und Bekanntmachung des Hinweises auf die Patenterteilung:
**24.03.2004 Patentblatt 2004/13**

(51) Int Cl.⁷: **A61K 7/42**, A61K 7/48

(21) Anmeldenummer: **98123264.8**

(22) Anmeldetag: **07.12.1998**

# (54) Verwendung von Bienenwachs zur Verstärkung der UV-A-Schutzleistung kosmetischer oder dermatologischer Formulierungen

Use of beeswax to increase the UVA protection factor of cosmetic or dermatologic sunscreens

Utilisation de la cire d' abeilles pour augmenter l' indice de protection UVA des compositions cosmétiques et dermatologiques photoprotectrices

(84) Benannte Vertragsstaaten:
**DE ES FR GB IT SE**

(30) Priorität: **18.12.1997 DE 19756376**

(43) Veröffentlichungstag der Anmeldung:
**18.08.1999 Patentblatt 1999/33**

(73) Patentinhaber: **Beiersdorf Aktiengesellschaft
20245 Hamburg (DE)**

(72) Erfinder:
- **Gers-Barlag, Heinrich, Dr.
25495 Kummerfeld (DE)**
- **Kröpke, Rainer
22869 Schenefeld (DE)**
- **Uhlmann, Beate, Dr.
22453 Hamburg (DE)**

(56) Entgegenhaltungen:
**WO-A-94/28867 WO-A-95/28912**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 935 959 B1

## Beschreibung

Die vorliegende Erfindung betrifft kosmetische und dermatologische Formulierungen, insbesondere kosmetische und dermatologische Lichtschutzformulierungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Vorbeugender Schutz gegen UV-A-Strahlen, beispielsweise durch Auftrag von Lichtschutzfiltersubstanzen in Form einer kosmetischen oder dermatologischen Formulierung auf die Haut, ist daher von grundsätzlicher Wichtigkeit.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Für die Dosierung der Substanzen in den fertigen Formulierungen können die Extinktionswerte allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Homschicht der Haut verteilt ist.

Zur Prüfung der UV-A-Schutzleistung wird üblicherweise die IPD-Methode verwendet (IPD ≡ immediate pigment darkening). Hierbei wird - ähnlich der Bestimmung des Lichtschutzfaktors - ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Die Einsatzkonzentration bekannter Lichtschutzfiltersubstanzen, die insbesondere auch im UV-A-Bereich eine hohe Filterwirkung zeigen, ist häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Da Lichtschutzfiltersubstanzen in der Regel kostspielig sind und da manche Lichtschutzfiltersubstanzen zudem schwierig in höheren Konzentrationen in kosmetische oder dermatologische Zubereitungen einzuarbeiten sind, war es Aufgabe der Erfindung, auf einfache und preiswerte Weise zu Zubereitungen zu gelangen, welche bei ungewöhnlich niedrigen Konzentrationen an herkömmlichen UV-A-Lichtschutzfiltersubstanzen dennoch eine akzeptable oder sogar hohe UV-A-Schutzleistung erreichen.

Es war indes überraschend und für den Fachmann nicht vorauszusehen, daß die Verwendung von Bienenwachs zur Erhöhung der UV-A-Schutzleistung kosmetischer oder dermatologischer Zubereitungen, enthaltend mindestens eine übliche UV-A-Filtersubstanz und/oder eine Breitbandfiltersubstanz, die auch gegen UV-Strahlung mit einer Wellenlänge, die größer als 335 nm ist, Schutz gewährt, den Nachteilen des Standes der Technik abhelfen würde.

Bienenwachs ist ein Ausscheidungsprodukt aus Drüsen der Honigbienen, das diese zum Bauen der Honigwaben verwenden. Gelbes (Cera flava), braunes oder rotes sogenanntes Rohwachs ist beispielsweise erhältlich, indem man die vom Honig durch Ausschleudern befreiten Waben schmilzt, die Schmelze (Schmp. 60-82° C) von festen Verunreinigungen trennt und das so erhaltene Rohwachs erstarren läßt. Das Rohwachs kann durch Behandlung mit Oxidationsmitteln vollkommen weiß gebleicht werden (Cera alba).

Bienenwachs besteht zu ca. 70 % aus einem Myricin genannten Gemisch von etwa 70 komplexen Wachsestern von $C_{16}$- bis $C_{36}$-Säuren und $C_{24}$- bis $C_{36}$-Alkoholen, zu 13 bis 14 % aus normalen Fettsäuren und Hydroxyfettsäuren sowie zu 10 bis 14 % aus Kohlenwasserstoffen.

Die Wachsester enthalten als Alkohol-Komponente vor allem Myricylalkohol $CH_3(CH_2)_{28}CH_2OH$, welcher insbesondere mit Palmitin- und Cerotinsäure zu Myricylpalmitat

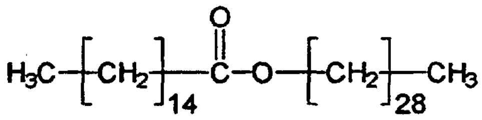

und Myricylcerotinat

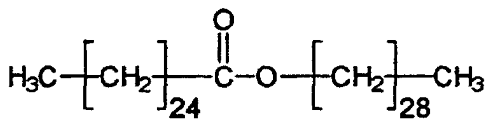

verestert ist.

Das Säuregemisch, auch Cerin genannt, ist in Alkohol leicht löslich und besteht vornehmlich aus Cerotinsäure $CH_3(CH_2)_{24}COOH$, Melissinsäure $CH_3(CH_2)_{28}COOH$ und Lignocerinsäure $CH_3(CH_2)_{22}COOH$.

Bienenwachs ist auch heute noch in kosmetischen oder dermatologischen Präparaten, Salben, Ceraten und Suppositorien zum Teil tragender Bestandteil, Konsistenzgeber und Verseifungsmittel. Gleichwohl konnte der Stand der Technik nicht den Weg zur vorliegenden Erfindung ebnen.

Erfindungsgemäß enthalten kosmetische oder dermatologische Zubereitungen vorteilhaft 0,1 bis 5 Gew.-%, insbesondere 0,5 bis 2,5 Gew.-% Bienenwachs.

Vorteilhafte UV-A-Filtersubstanzen im Sinne der vorliegenden Erfindung sind Dibenzoylmethanderivate, insbesondere 5-Isopropyldibenzoylmethan (CAS-Nr. 63250-25-9), welches sich durch die Struktur

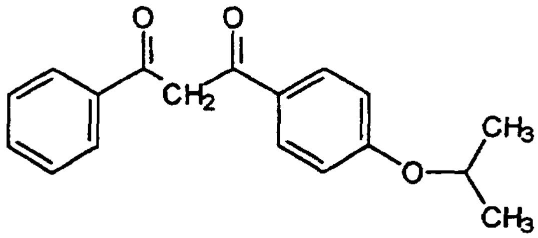

auszeichnet und von Merck unter der Marke Eusolex® 8020 verkauft wird, und/oder das 4-(tert.-Butyl)-4'-methoxydibenzoylmethan (CAS-Nr. 70356-09-1), welches sich durch die Struktur

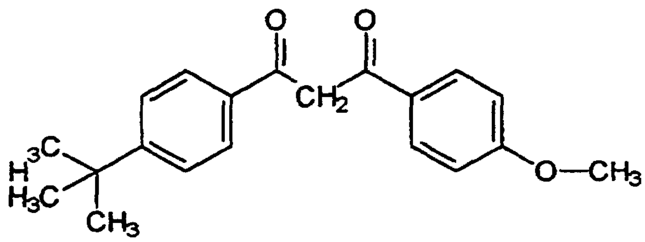

auszeichnet und von Givaudan unter der Marke Parsol® 1789 verkauft wird.

Weitere vorteilhafte UV-A-Filtersubstanzen sind die Phenylen-1,4-bis-(2-benzimidazyl)-3,3'-5,5'-tetrasulfonsäure:

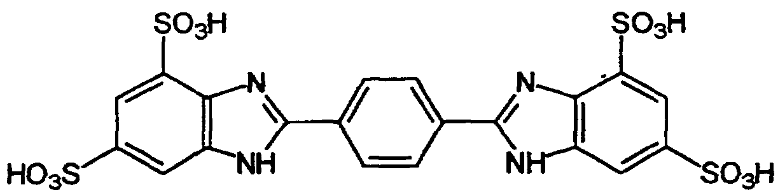

und ihre Salze, besonders die entsprechenden Natrium-, Kalium- oder Triethanolammonium-Salze, insbesondere das Phenylen-1,4-bis-(2-benzimidazyl)-3,3'-5,5'tetrasulfonsäure-bis-natriumsalz:

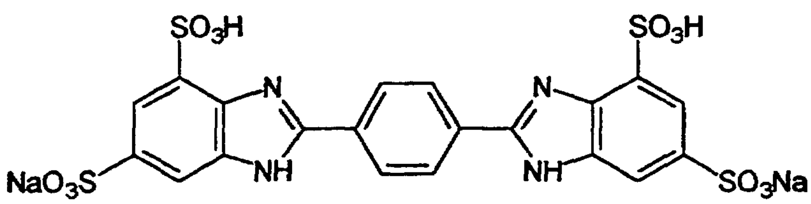

sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-Sulfonsäure) bezeichnet wird und sich durch die folgende Struktur auszeichnet:

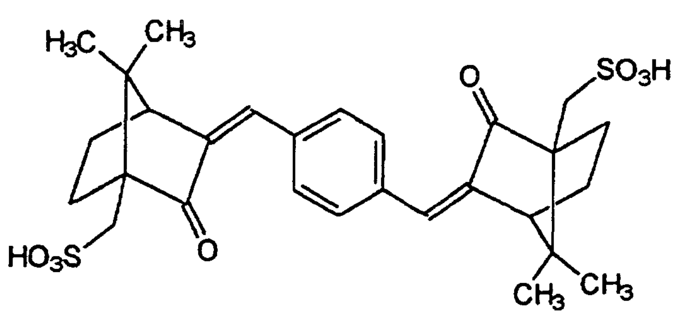

Vorteilhafte UV-A-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter sind beispielsweise Bis-Resorcinyltriazinderivate mit der folgenden Struktur:

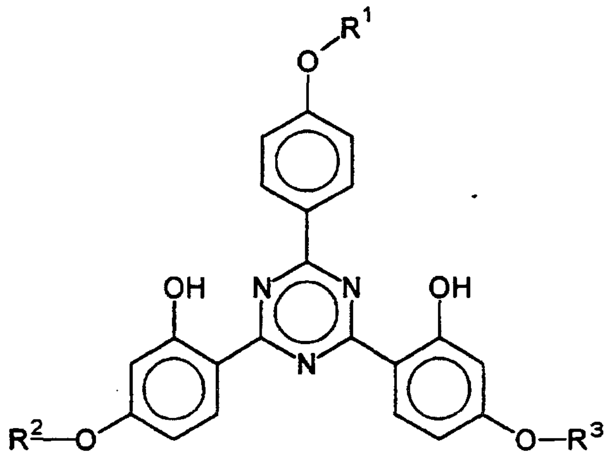

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethylhexyloxy)-2-hydroxy]-phenyl}-6-(4-methoxyphenyl)-1,3,5-triazin, welches durch folgende Struktur gekennzeichnet ist:

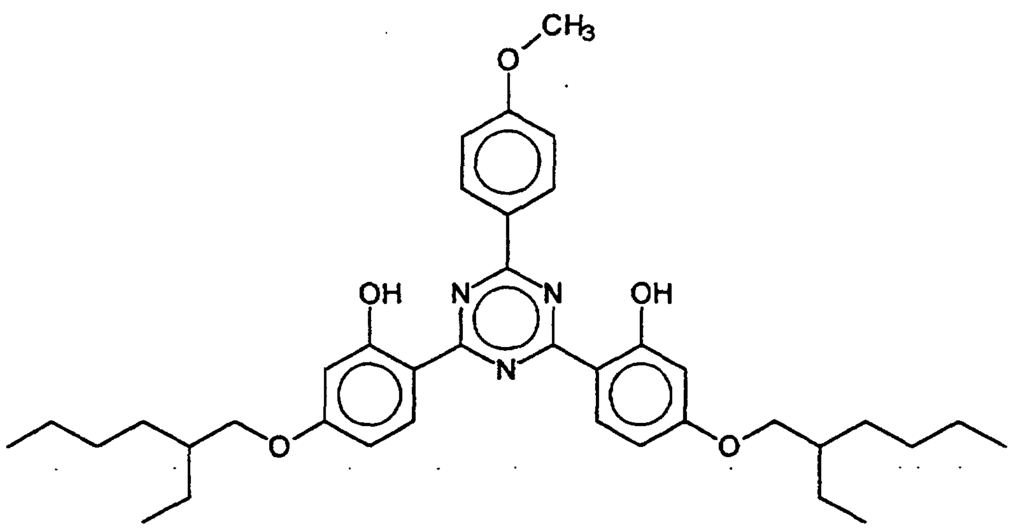

Kosmetische und dermatologische Formulierungen im Sinne der Erfindung enthalten einen oder mehrere übliche UV-A-Filter als Einzelsubstanzen oder in beliebigen Gemischen untereinander, in der Lipidphase und/oder in der wäßrigen Phase.

Besonders bevorzugt ist die Verwendung von Bienenwachs zur Erhöhung der UV-A-Schutzleistung von Dibenzoylmethanderivaten, insbesondere von 5-Isopropyldibenzoylmethan und/oder 4-(tert.-Butyl)-4'-methoxydibenzoylmethan. Erstaunlicherweise steigert die Verwendung von Bienenwachs die UV-A-Schutzleistung von Dibenzoylmethanderivaten erheblich.

Die Gesamtmenge an UV-A-Filtersubstanzen in den fertigen kosmetischen oder dermatologischen Zubereitungen wird vorteilhaft aus dem Bereich von 0,1 bis 30 Gew.-%, bevorzugt 0,1 bis 10,0 Gew.-%, insbesondere 0,5 bis 5,0 Gew.-% gewählt, bezogen auf das Gesamtgewicht der Zubereitungen.

Erfindungsgemäße kosmetische und dermatologische Zubereitungen enthalten femer vorteilhaft, wenngleich nicht zwingend, anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Derlei Pigmente sind im Sinne der Erfindung als UV-Filtersubstanzen anzusehen.

Die anorganischen Pigmente liegen erfindungsgemäß vorteilhaft in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 3314 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0, insbesondere 0,5 - 6,0 Gew.-% gewählt, bezogen auf das Gesamtgewicht der Zubereitungen.

Die erfindungsgemäßen kosmetischen und/oder dermatologischen Formulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen. Entsprechend können die erfindungsgemäßen Zubereitungen, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zubereitungen als Grundlage für pharmazeutische Formulierungen zu verwenden. Bevorzugt sind insbesondere solche kosmetischen und dermatologischen Zubereitungen, die in der Form eines Hautpflege- bzw. Schminkproduktes vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UV-A-Filter und/oder mindestens einen UV-B-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die jeweils einzusetzenden Mengen an kosmetischen oder dermatologischen Hilfs- und Trägerstoffen und Parfüm können in Abhängigkeit von der Art des jeweiligen Produktes vom Fachmann durch einfaches Ausprobieren leicht ermittelt werden.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Camosin und deren Derivate (z.B. Anserin), Carotinoide, Carotine (z.B. $\alpha$-Carotin, $\beta$-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis $\mu$mol/kg), ferner (Metall)-Chelatoren (z.B. $\alpha$-Hydroxyfettsäuren, Palmitinsäure, Phytinsäure, Lactoferrin), $\alpha$-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß wird die UV-A-Schutzleistung durch die Verwendung von Bienenwachs in kosmetischen oder dermatologischen Zubereitungen, enthaltend übliche UV-A-Filter und/oder UV-B-Filter, sei es als Einzelsubstanzen oder in beliebigen Gemischen untereinander, in der Lipidphase und/oder in der wäßrigen Phase erheblich gesteigert.

Vorteilhaft können die erfindungsgemäßen Lichtschutzformulierungen Substanzen enthalten, die UV-Strahlung im UV-B-Bereich absorbieren, wobei die Gesamtmenge der Filtersubstanzen z.B. 0,1 Gew.-% bis 30 Gew.-%, vorzugsweise 0,5 bis 20 Gew.-%, insbesondere 1 bis 10 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die UV-B-Filter können öllöslich oder wasserlöslich sein. Vorteilhafte öllösliche UV-B-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

Vorteilhafte wasserlösliche UV-B-Filtersubstanzen sind z.B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bornylidenmethyl)sulfonsäure und deren Salze.

Die Liste der genannten UV-B-Filter, die zusätzlich im Sinne der vorliegenden Erfindung eingesetzt werden können, soll selbstverständlich nicht limitierend sein.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Gesamtmenge an wasserlöslichen UV-Filtersubstanzen in den fertigen kosmetischen oder dermatologischen Zubereitungen, beispielsweise an 2-Phenylbenzimidazol-5-sulfonsäure bzw. deren Salzen und/oder 2-Hydroxy-4-methoxybenzophenon-5-sulfonsäure bzw. deren Salzen und/oder 4-(2-Oxo-3-bornylidenmethyl)benzolsulfonsäure bzw. deren Salzen und/oder 2-Methyl-5-(2-oxo-3-bornylidenmethyl)benzolsulfonsäure bzw. deren Salzen und/oder Benzol-1,4-di(2-oxo-3-bornylidenmethyl)-10-Sulfonsäure bzw. deren Salzen, wird vorteilhaft aus dem Bereich von 0,1 - 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Die Gesamtmenge an öllöslichen UV-Filtersubstanzen in den fertigen kosmetischen oder dermatologischen Zubereitungen, beispielsweise an 4,4',4''-(1,3,5-Triazin-2,4,6-triyltriimino)-tris-benzoesäure-tris(2-ethylhexylester) und/oder 4-(tert.-Butyl)-4'-methoxydibenzoylmethan und/oder 4-Methylbenzylidencampher, wird vorteilhaft aus dem

Bereich von 0,1 10,0 Gew.-%, bevorzugt 0,5 - 6,0 Gew.-% gewählt, bezogen auf das Gesamtgewicht der Zubereitungen, falls die Gegenwart dieser Substanzen erwünscht ist.

Die Gesamtmenge an 2-Ethylhexyl-p-methoxy-cinnamat in den fertigen kosmetischen oder dermatologischen Zubereitungen wird, falls die Gegenwart dieser Substanz erwünscht ist, vorteilhaft aus dem Bereich von 0,1 15,0 Gew.-%, bevorzugt 0,5 - 7,5 Gew.-% gewählt, bezogen auf das Gesamtgewicht der Zubereitungen.

Noch eine weiterere erfindungsgemäß vorteilhaft zu verwendende zusätzliche Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

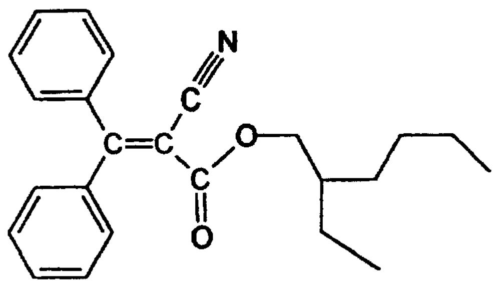

Die Gesamtmenge an Ethylhexyl-2-cyano-3,3-diphenylacrylat in den fertigen kosmetischen oder dermatologischen Zubereitungen wird, falls die Gegenwart dieser Substanz erwünscht ist, vorteilhaft aus dem Bereich von 0,1 - 15,0 Gew.-%, bevorzugt 0,5 - 10,0 Gew.-% gewählt, bezogen auf das Gesamtgewicht der Zubereitungen.

Ferner kann gegebenenfalls von Vorteil sein, erfindungsgemäß weitere UV-A- und/oder UV-B-Filtern in kosmetische oder dermatologisch Zubereitungen einzuarbeiten, beispielsweise bestimmte Salicylsäurederivate wie 4-Isopropylbenzylsalicylat, 2-Ethylhexylsalicylat (=Octylsalicylat), Homomenthylsalicylat.

Die Gesamtmenge an einem oder mehreren Salicylsäurederivaten in den fertigen kosmetischen oder dermatologischen Zubereitungen wird vorteilhaft aus dem Bereich von 0,1 - 15,0 Gew.-%, bevorzugt 0,5 - 8,0 Gew.-% gewählt, bezogen auf das Gesamtgewicht der Zubereitungen. Wenn Ethylhexylsalicylat gewählt wird, ist es von Vorteil, dessen Gesamtmenge aus dem Bereich von 0,1 - 5,0 Gew.-%, bevorzugt 0,5 - 2,5 Gew.-% zu wählen. Wenn Homomenthylsalicylat gewählt wird, ist es von Vorteil, dessen Gesamtmenge aus dem Bereich von 0,1 - 10,0 Gew.-%, bevorzugt 0,5 - 5,0 Gew.-% zu wählen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **Beispiel 1** | |
|---|---|
| O/W-Lotion | |
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |
| Parsol® 1789[1] | 3,00 |
| Bienenwachs | 1,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

| **Beispiel 2** | |
|---|---|
| Hydrodispersionsgel | |
| | Gew.-% |
| Pemulen® TR-1[2] | 0,50 |
| Ethanol | 3,50 |
| Glycerin | 3,00 |
| Dimethicon | 1,50 |
| Octyldodecanol | 0,50 |
| Capryl-/Caprinsäuretriglycerid | 5,00 |
| Bienenwachs | 2,00 |
| Parsol® 1789[1] | 5,00 |
| Carbomer | 0,20 |
| Natriumhydroxid (45%ig) | 0,55 |
| Konservierungsmittel | q.s. |
| Parfum | q.s. |
| Wasser, demin. | ad 100,00 |

| **Beispiel 3** | |
|---|---|
| O/W-Creme | |
| | Gew.-% |
| Glycerylstearat SE | 3,50 |
| Stearinsäure | 3,50 |
| Butylenglykol | 5,00 |
| Cetearylalkohol | 3,00 |
| $C_{12}$-$C_{15}$ Alkyl Benzoate | 10,0 |
| Parsol® 1789[1] | 4,00 |
| Uvinul®T150[3] | 4,00 |
| Bienenwachs | 3,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

| **Beispiel 4** | |
|---|---|
| W/O-Lotion | |
| | Gew.-% |
| Dehymuls® PGPH[4] | 3,50 |
| Lameform® TGI[5] | 3,50 |
| Butylenglykol | 5,00 |
| Ceresin | 3,00 |
| $C_{12}$-$C_{15}$ Alkyl Benzoate | 10,0 |
| Uvinul®T150[3] | 4,00 |
| Parsol® 1789[1] | 4,00 |
| Bienenwachs | 2,00 |
| Vaseline | 2,00 |

(fortgesetzt)

| Beispiel 4 | |
|---|---|
| W/O-Lotion | |
| | Gew.-% |
| Natriumhydroxid (45%ig) | 0,35 |
| Konservierungsmittel | q.s. |
| Parfum | q.s. |
| Wasser, demin. | ad 100,00 |

| Beispiel 5 | |
|---|---|
| O/W-Lotion | |
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |
| Uvinul®T150[3] | 3,00 |
| Bienenwachs | 1,00 |
| Parsol® 1789[1] | 2,00 |
| Eusolex® 6300[6] | 1,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

| Beispiel 6 | |
|---|---|
| Hydrodispersionsgel | |
| | Gew.-% |
| Pemulen® TR-1[2] | 0,50 |
| Ethanol | 3,50 |
| Glycerin | 3,00 |
| Dimethicon | 1,50 |
| Octyldodecanol | 0,50 |
| Capryl-/Caprinsäuretriglycerid | 5,00 |
| Bienenwachs | 2,00 |
| Uvinul®T150[1] | 5,00 |
| Parsol® 1789[1] | 2,00 |
| Eusolex® 6300[6] | 1,00 |
| Carbomer | 0,20 |
| Natriumhydroxid (45%ig) | 0,55 |
| Konservierungsmittel | q.s. |
| Parfum | q.s. |
| Wasser, demin. | ad 100,00 |

| **Beispiel 7** | |
|---|---|
| O/W-Creme | |
| | Gew.-% |
| Glycerylstearat SE | 3,50 |
| Stearinsäure | 3,50 |
| Butylenglykol | 5,00 |
| Cetearylalkohol | 3,00 |
| $C_{12}$-$C_{15}$ Alkyl Benzoate | 10,0 |
| Uvinul®T150[1] | 4,00 |
| CGF 1607[7] | 2,00 |
| Eusolex® 6300[6] | 1,00 |
| Bienenwachs | 3,00 |
| Carbomer | 0,20 |
| Natriumhydroxid (45%ig) | 0,35 |
| Konservierungsmittel | q.s. |
| Parfum | q.s. |
| Wasser, demin. | ad 100,00 |

| **Beispiel 8** | |
|---|---|
| W/O-Lotion | |
| | Gew.-% |
| Dehymuls® PGPH[4] | 3,50 |
| Lameform® TGI[5] | 3,50 |
| Butylenglykol | 5,00 |
| Ceresin | 3,00 |
| $C_{12}$-$C_{15}$ Alkyl Benzoate | 10,0 |
| Uvinul®T150[1] | 4,00 |
| Parsol® 1789[1] | 2,00 |
| Eusolex® 6300[6] | 1,00 |
| Titandioxid | 2,00 |
| Bienenwachs | 2,00 |
| Vaseline | 2,00 |
| Natriumhydroxid (45%ig) | 0,35 |
| Konservierungsmittel | q.s. |
| Parfum | q.s. |
| Wasser, demin. | ad 100,00 |

| **Beispiel 9** | |
|---|---|
| O/W-Lotion | |
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |

(fortgesetzt)

| Beispiel 9 | |
|---|---|
| O/W-Lotion | |
| | Gew.-% |
| Parsol® 1789[1] | 3,00 |
| Bienenwachs | 1,00 |
| CGF 1607[7] | 4,50 |
| Carbomer | 0,20 |
| Natriumhydroxid (45%ig) | 0,20 |
| Konservierungsmittel | q.s. |
| Parfum | q.s. |
| Wasser, demin. | ad 100,00 |

| Beispiel 10 | |
|---|---|
| O/W-Lotion | |
| | Gew.-% |
| Glycerylstearat SE | 3,50 |
| Stearinsäure | 1,80 |
| Glycerin | 3,00 |
| Cetearylalkohol | 0,50 |
| Octyldodecanol | 7,00 |
| Dicaprylylether | 8,00 |
| 2-Ethylhexylsalicylat | 5,00 |
| Bienenwachs | 2,00 |
| Parsol® 1789[1] | 3,00 |
| 2-Hydroxy-4-methoxybenzophenon | 5,00 |
| Carbomer | 0,20 |
| Natriumhydroxid (45%ig) | 0,20 |
| Konservierungsmittel | q.s. |
| Parfum | q.s. |
| Wasser, demin. | ad 100,00 |

[1] 4-(tert.-Butyl)-4'-methoxydibenzoylmethan
[2] Copolymer aus $C_{10-30}$ Alkylacrylaten und einem oder mehreren Monomeren der Acrylsäure,der Methacrylsäure oder deren Ester, die kreuzvernetzt sind mit einem Allylether der Saccharose oder einem Allylether des Pentaerythrit (CTFA-Bez.: Acrylates/C 10-30 Alkyl Acrylate Crosspolymer)
[3] 4,4',4"-(1,3,5-Triazin-2,4,6-triyltriimino)-tris-benzoesäure-tris(2-ethylhexylester)
[4] Triglycerindiisostearat
[5] Polyglycerinpolyhydroxystearat
[6] 3-(4-Methylbenzyliden)campher
[7] 2,4-Bis-{[4-(2-Ethyl-hexyloxy)-2-hydroxy]-phenyl}-6-(4-methoxyphenyl)-1,3,5-triazin

## Patentansprüche

1. Verwendung von Bienenwachs zur Erhöhung der UV-A-Schutzleistung kosmetischer oder dermatologischer Zubereitungen, enthaltend mindestens eine übliche UV-A-Filtersubstanz und/oder eine Breitbandfiltersubstanz, die auch gegen UV-Strahlung mit einer Wellenlänge, die größer als 335 nm ist, Schutz gewährt.

2. Verwendung nach Anspruch 1, **dadurch gekennzeichnet, daß** die kosmetischen oder dermatologischen Lichtschutzzubereitungen 0,1 bis 20 Gew.-% an Bienenwachs enthalten.

3. Verwendung nach Anspruch 1, **dadurch gekennzeichnet, daß** die kosmetischen oder dermatologischen Lichtschutzzubereitungen 0,5 bis 10 Gew.-% Bienenwachs enthalten.

4. Verwendung nach Anspruch 1, **dadurch gekennzeichnet, daß** die kosmetischen oder dermatologischen Lichtschutzzubereitungen als UV-A-Filtersubstanz ein oder mehrere fettlösliche UV-A-Filtersubstanzen, besonders Dibenzoylmethanderivate, insbesondere 5-Isopropyldibenzoylmethan und/oder 4-(tert.-Butyl)-4'-methoxydibenzoylmethan, enthalten.

5. Verwendung nach Anspruch 1, **dadurch gekennzeichnet, daß** die kosmetischen oder dermatologischen Lichtschutzzubereitungen als UV-A-Filtersubstanz ein oder mehrere wasserlösliche UV-A-Filtersubstanzen, besonders wasserlösliche UV-A-Filtersubstanzen gewählt aus der Gruppe Phenylen-1,4-bis-(2-benzimidazyl)-3,3'-5,5'-tetrasulfonsäure und/oder 1,4-di(2-oxo-10-Sulfo-3-bornylidenmethyl)-Benzol und/oder ihre Salze, besonders die entsprechenden Natrium-, Kalium- oder Triethanolammonium-Salze und/oder die entprechenden 10-Sulfato-verbindungen, insbesondere das Phenylen-1,4-bis-(2-benzimidazyl)-3,3'-5,5'-tetrasulfonsäure-bis-natriumsalz, enthalten.

6. Verwendung nach Anspruch 1, **dadurch gekennzeichnet, daß** als UV-A-Filtersubstanz ein Breitbandfilter aus der Gruppe der Bis-Resorcinyltriazinderivate gewählt wird, insbesondere das 2,4-Bis-{[4-(2-Ethyl-hexyloxy)-2-hydroxy]-phenyl}-6-(4-methoxyphenyl)-1,3,5-triazin.

## Claims

1. Use of beeswax for increasing the UV-A protection performance of cosmetic or dermatological preparations comprising at least one customary UV-A filter substance and/or one broadband filter substance which also affords protection against UV radiation with a wavelength greater than 335 nm.

2. Use according to Claim 1, **characterized in that** the cosmetic or dermatological light protection preparations comprise 0.1 to 20% by weight of beeswax.

3. Use according to Claim 1, **characterized in that** the cosmetic or dermatological light protection preparations comprise 0.5 to 10% by weight of beeswax.

4. Use according to Claim 1, **characterized in that** the cosmetic or dermatological light protection preparations comprise, as UV-A filter substance, one or more fat-soluble UV-A filter substances, particularly dibenzoylmethane derivatives, in particular 5-isopropyldibenzoylmethane and/or 4-(tert-butyl)-4'-methoxydibenzoylmethane.

5. Use according to Claim 1, **characterized in that** the cosmetic or dermatological light protection preparations comprise, as UV-A filter substance, one or more water-soluble UV-A filter substances, particularly water-soluble UV-A-filter substances chosen from the group consisting of phenylene-1,4-bis(2-benzimidazyl)-3,3'-5,5'-tetrasulfonic acid and/or 1,4-di(2-oxo-10-sulfo-3-bornylidenemethyl)benzene and/or its salts, particularly the corresponding sodium, postassium and triethanolammonium salts and/or the corresponding 10-sulfato compounds, in particular the phenylen-1-4-bis(3-benzimidazyl)-3,3'-5,5'-tetrasulfonic acid bis-sodium salt.

6. Use according to Claim 1, **characterized in that** the UV-A filter substance chosen is a broadband filter from the group of bisresorcinyltriazine derivatives, in particular 2,4-bis{[4-(ethylhexyloxy)-2-hyroxyl]phenyl)-6-(4-methoxyphenyl)-1,3,5-triazine.

## Revendications

1. Utilisation de cire d'abeille pour accroître le pouvoir protecteur contre les UV-A de préparations cosmétiques ou dermatologiques contenant au moins une substance usuelle filtrant les UV-A et/ou une substance filtrante à large bande qui assure la protection également contre un rayonnement UV ayant une longueur d'onde supérieure à 335 nm.

2. Utilisation selon la revendication 1, **caractérisée en ce que** les préparations photoprotectrices cosmétiques ou dermatologiques contiennent de 0,1 à 20 % en poids de cire d'abeille.

3. Utilisation selon la revendication 1, **caractérisée en ce que** les préparations photoprotectrices cosmétiques ou dermatologiques contiennent de 0,5 à 10 % en poids de cire d'abeille.

4. Utilisation selon la revendication 1, **caractérisée en ce que** les préparations photoprotectrices cosmétiques ou dermatologiques contiennent en tant que substance filtrant les UV-A un ou plusieurs filtres UV-A liposolubles, en particulier des dérivés de dibenzoylméthane, notamment le 5-isopropyldibenzoylméthane et/ou le 4-(tert-butyl)-4'-méthoxydibenzoylméthane.

5. Utilisation selon la revendication 1, **caractérisée en ce que** les préparations photoprotectrices cosmétiques ou dermatologiques contiennent en tant que substance filtrant les UV-A un ou plusieurs filtres UV-A hydrosolubles, en particulier des filtres UV-A hydrosolubles choisis dans l'ensemble constitué par l'acide phénylène-1,4-bis(2-benzimidazyl)-3,3'-5,5'-tétrasulfonique et/ou le 1,4-di(2-oxo-10-sulfo-3-bornylidèneméthyl)benzène et/ou leurs sels, en particulier les sels de sodium, potassium ou triéthanolammonium correspondants et/ou les composés 10-sulfato correspondants, notamment le phénylène-1,4-bis(2-benzimidazyl)-3,3'-5,5'-tétrasulfonate disodique.

6. Utilisation selon la revendication 1, **caractérisée en ce que** comme substance filtrant les UV-A on choisit un filtre à large bande choisi dans le groupe des dérivés de bis-résorcinyltriazine, en particulier la 2,4-bis{[4-(2-éthyl-hexyloxy)-2-hydroxy]-phényl}-6-(4-méthoxyphényl)-1,3,5-triazine.